# EUROPEAN PATENT SPECIFICATION

(11) **EP 4 463 177 B1**
(45) Date of publication and mention of the grant of the patent: **14.05.2025**
(21) Application number: 24703801.1
(22) Date of filing: 08.02.2024
(51) Int. Cl.: A61K 38/39, A23L 33/28, A61P 25/20

(54) **USE OF COLLAGEN HYDROLYSATE AS AN ACTIVE INGREDIENT IN AMELIORATING SLEEP**
VERWENDUNG VON KOLLAGENHYDROLYSAT ALS WIRKSTOFF ZUR VERBESSERUNG DES SCHLAFES
UTILISATION D'UN HYDROLYSAT DE COLLAGÈNE EN TANT QUE PRINCIPE ACTIF À DES FINS D'AMÉLIORATION DU SOMMEIL

(30) Priority: 08.02.2023 BE 202305091
(43) Date of publication of application: 20.11.2024
(73) Proprietor: Rousselot BV, 9000 Gent (BE)
(72) Inventor: VIRGILIO, Nicolina, 9000 GENT (BE); PRAWITT, Janne, 9000 GENT (BE)
(74) Representative: EP&C
(86) International application number: PCT/EP2024/053169
(87) International publication number: WO 2024/165665

(56) References cited:
- WO-A1-2021/156400
- US-A1- 2018 021 411
- LEÓN-LÓPEZ ARELY ET AL: "Hydrolyzed Collagen-Sources and Applications", vol. 24, no. 22, 7 November 2019 (2019-11-07), pages 4031, XP055903004, Retrieved from the Internet <URL:https://www.ncbi.nlm.nih.gov/labs/pmc/articles/PMC6891674/pdf/molecules-24-04031.pdf> DOI: 10.3390/molecules24224031

## Description

### TECHNICAL FIELD

The current invention pertains to an active ingredient in ameliorating sleep. The active ingredient can be administered as oral supplement.

### BACKGROUND OF THE INVENTION

Sleep is an important aspect of health and quality of life and is part of a basic physiologic rhythm that is characterized in humans by three states: wakefulness, non-rapid eye movement (NREM) sleep, and rapid eye movement (REM) sleep.

Sleep problems are an increasing problem worldwide. Impairments in sleep can arise as disturbances of usual sleep patterns and/or sleep-related cognitive behaviours. It is thought that 30 - 50% of individuals awake too frequently during the night.

It is furthermore estimated that 30-50% of individuals wake up in the morning feeling un-refreshed or experience lowered cognitive performance, which appears associated with a sleep deprivation and/or poor sleep quality. The impairment in cognitive function is the most evident in terms of attention, concentration, and other cognitive domains such as the working memory, reaction time, susceptibility to cognitive interference, episodic memory, and impulse inhibition.

Sleep and sleep quality can thus be characterized as the overall experience considering various parameters such as the time needed to fall asleep (sleep onset), either or not waking frequently during the night (fragmented sleep, sleep awakenings), waking too early (premature final awakening), waking up feeling un-refreshed, daytime sleepiness, and waking up with cognitive impairments.

Depending on the severity and duration, an impairment in sleep can manifest as either non-pathological (i.e. a non-medical condition) or pathological (i.e. as a medical condition).

An improvement in sleep, also in non-pathological context, can lead to improved general well-being, health, and performance. For example, it is known that acute sleep deprivation and partial sleep restriction can evoke stress associated with prolonged awakening and that reductions in sleep quality impact emotional volatility and irritability, and leading to lower positive mood. It has furthermore been found that improvement of sleep accelerates learning ability (Stickgold et al. Nat Neurosci. 2000 December; 3(12):1237-8), improves activity of the immune system and the host defence (Irwin et al. FASEB J. 1996 April; 10(5):643-53.), and decreases the risk of glucose intolerance and diabetes (Gottlieb et al. Arch Intern Med. 2005 Apr. 25; 165(8):863-7). Thus, there can be different reasons why (further) improvement of sleep and sleep quality is beneficial, even in absence of a sleep-related medical condition.

An improvement in sleep can also be necessitated by medical conditions (i.e. sleep disorders). Insomnia is the most common sleep disorder and is typically classified as being chronic insomnia (e.g. greater than three weeks in duration), short-term insomnia (e.g. one to three weeks in duration), or transient insomnia (e.g. less than one week in duration) (Roth, Int. J. Clin. Pract. 2001; (Suppl.):3-8). Individuals suffering insomnia can also be classified based on when their sleep difficulty most often occurs, i.e. as (1) sleep onset insomnia (difficulty in falling asleep); (2) sleep maintenance insomnia (difficulty staying asleep); and (3) terminal insomnia (early-morning awakenings coupled with an inability to return to sleep).

Several ingredients are administered enhance sleep. For example, benzodiazepines are frequently used as sleep-enhancers and exert their pharmacological actions by interacting with the benzodiazepine binding sites associated with the gamma amino butyric acid (GABA) receptor. GABA is a main inhibitory neurotransmitter of the central nervous system. GABA plays a role in regulating sleep, anxiety, convulsion, depression, psychosis, cognition, and the like, states in mammals. Well-known benzodiazepines include diazepam (Valium), lorazepam (Temesta), clonazepam (Rivotril) and alprazolam (Xanax). GABA's stress-reducing, and sleep enhancing effects have also been established and GABA is therefore found in various sleep-enhancing compositions. Other well-known active ingredients used to improve sleep include sedative antidepressants, antipsychotics, antihistamines, non-benzodiazepine GABA modulators, melatonin receptor agonist, serotonin receptor agonists, and glucocorticoid inhibitors (e.g. phosphatidylserine).

US 2018/021411A1 discloses a composition suitable for the treatment of sleep disorders comprising i) collagen peptides or gelatin peptides, ii) L-theanine and iii) lactucopicrin, deoxylactucopicrin, or another lactucopicrin derivative. WO2021/156400A1 discloses a composition suitable for improving sleep quality comprising collagen peptides, GABA, L-theanine, 5-HTP, L-tryptophan and magnesium.

As a limitation of the current sleep-enhancers, many of them have very undesirable side effects, including one or more of abnormal thinking and behavioural changes, hallucinations, complex behaviours such as sleep driving, amnesia, anxiety, suicidal thinking, withdrawal symptoms, depressant effects, impaired motor or cognitive performance, bad taste, headaches, parasomnias such as sleep walking, anaphylactic and anaphylactoid reactions and/or weight gain.

In addition, existing sleep-enhancers may help improve sleep onset or sleep time, but may not satisfactorily improve, or may even have side effects on, overall sleep quality. For example, although benzodiazepines and other GABA modulators typically can promote sleep onset, but they nevertheless have limited effectiveness in promoting a full night's sleep, have no benefit in reducing early awakenings, and typically even worsen the behavioural and cognitive functions in the morning. Classic benzodiazepines typically are also known to reduce slow wave sleep (SWS) and rapid eye movement (REM) sleep.

It is an object of the present invention to provide a safer and improved sleep enhancer.

### SUMMARY OF THE INVENTION

The invention is set out as in the appended claims.

The present inventors found that intake of collagen hydrolysate improves the overall sleep. Intake of collagen hydrolysate as the only active ingredient led to improved overall sleep compared to intake of collagen hydrolysate in combination with well-known sleep-enhancing compounds, such as gamma-aminobutyric acid, L-Theanine, 5-Hydroxytryptophan, and/or L-Tryptophan.

Without being bound by theory, it is found that collagen hydrolysate has an inherently strong sleep-enhancing effect. The sleep-enhancing effect of collagen hydrolysate appears however counteracted by known sleep-enhancing compounds, such as gamma-aminobutyric acid (GABA), L-Theanine, 5-Hydroxytryptophan, and/or (L-)Tryptophan, but in particular GABA.

The findings by the present inventors are surprising, because the existing belief is that collagen hydrolysate is not effective as sleep-enhancer when administered alone. Hence, in the art, collagen hydrolysate is combined with other sleep-enhancing compounds to have a sleep-enhancing effect. For example, US10568939B2 discloses that collagen peptide, when used alone, has no effect at all on sleep onset, duration, or quality, and therefore should be combined with well-known sleep compounds.

With the current invention, the present inventors have overcome an existing prejudice in the field.

It was found that collagen hydrolysate is particularly effective in improving sleep in individuals that are physically more active. Foremost, the overall sleep quality in athletes was remarkably improved by intake of collagen hydrolysate. Thus, the collagen hydrolysate is preferably taken by physically active individuals, in particular athletes.

It was furthermore found that intake of collagen hydrolysate led to measurable improvement in sleep already within one week. In comparison, the effect of sleep was delayed when collagen hydrolysate was combined with GABA. Thus, there is a faster benefit for collagen hydrolysate as sleep-enhancer when it is taken alone and without other sleep-enhancers.

It is found to be most beneficial in the context of the invention when the collagen hydrolysate is administered relatively close to bedtime, such as 4 hours within prior to bedtime, more preferably 2 hours within prior to bedtime (e.g. 2 h - 30 min prior to bedtime). Without being bound to theory, the present inventors found an optimal bioavailability of the collagen hydrolysate and its derived components after oral intake relatively close to bedtime, and which is associated with improved sleep (e.g. as compared to earlier oral intake).

The present inventors found that use of collagen hydrolysate without other well-known sleep-enhancing compounds is safe and leads to lowest complaints such as related to fatigue, mood, or psychomotor. In particular, use of collagen hydrolysate alone led to the highest neurocognitive performance after bedtime.

The use of collagen hydrolysate as sleep-enhancer in the present invention is generally considered safe and can be used for instance as daily supplement, both as a therapeutic or a non-therapeutic method (e.g. requiring no medical prescription).

In an aspect, the current disclosure describes a use of collagen hydrolysate as active ingredient in ameliorating sleep.

In an aspect, the current disclosure describes collagen hydrolysate for use as active ingredient in ameliorating a sleep disorder, such as selected from the group consisting of insomnia, restless legs syndrome, narcolepsy and sleep apnoea.

In an aspect, the current disclosure describes a composition suitable for ameliorating sleep, the composition comprising at least 97.5 wt.%, preferably 97.5 wt.% collagen hydrolysate as active ingredient and no more than 2.5 wt.%, preferably 2.5 wt.% of one or more further compounds modulating sleep, calculated on the active ingredients and/or the compounds capable of modulating sleep in the composition.

In an aspect, the current disclosure describes a sleep-ameliorating composition, consisting essentially of collagen hydrolysate and/or comprising essentially no further compound capable of modulating sleep.

### DETAILLED DESCRIPTION OF THE INVENTION

The use in the context of the current invention pertains to a use in ameliorating sleep.

In the art, the use of collagen hydrolysate in sleep-enhancing formulations is already known, but in combination with sleep-enhancing compounds, such as GABA receptor agonist and/or melatonin receptor agonists. As such, a consensus in the art is that collagen hydrolysate by itself cannot enhance sleep, but may modulate the activity or effectiveness of other sleep-enhancing compounds. The present inventors however found that overall improvement in sleep is highest when collagen hydrolysate is used as main or the only active ingredient to modulate sleep.

In the context of the current invention, the collagen hydrolysate is an active ingredient, more preferably an active ingredient in ameliorating (e.g. improving) sleep and/or ameliorating a sleep disorder.

In the context of the current invention, the term "active ingredient" is a component of a dosage form that performs a biological function when administered or induces or affects (e.g. enhances or inhibits) a physiological process in some manner. "Activity" is the ability to perform the function, or to induce or affect the process. Active ingredients are distinguishable from excipients, such as carriers, vehicles, diluents, lubricants, binders, disintegrants, fillers, and other formulating aids, and encapsulating or otherwise protective components. Compounds capable of modulating sleep (e.g. enhancing sleep) are examples of active ingredients. In the context of the current invention, the active ingredient may be an ingredient that is not a compound capable of modulating sleep, but rather instead for example an ingredient which has a positive effect on general health or performance.

### Collagen hydrolysate of the invention

The term "collagen" in the context of the current invention means an amino acid sequence comprising a repeating (Gly-X-Y) sequence, preferably comprising at least 1, 2, 3, 4, 5, 10, 20, 50, 100, 200, 300, or 400 sequences containing the sequence Gly-X-Y, where X and Y are an amino acid residue independently chosen from each other, but X and/or Y are more preferably proline. The "collagen" preferably has a sequence found in natural collagen in one or more animal species. In addition or alternatively, the "collagen" can mean a full-length sequence or fragment or subunit thereof of (native) collagen, preferably one or more of collagen types I - XXVII, more preferably one or even more of type I, II, III, V, or X collagen, even more preferably one or more of type I, II or III collagen. For example, the term "collagen" may refer to an alpha-1(I), alpha-2(I), alpha-1(ll) or alpha-1(lll) chain, or a fragment thereof. The term "collagen" encompasses the triple helix structure as formed by three subunits as present in native collagen.

The term "collagen hydrolysate" the context of the current invention means a mix of short chains of amino acids (di-, tri, oligopeptides, polypeptides) derived from (partial) hydrolysis from native (full-length) collagen, such as by enzymatic hydrolysis. The degree of hydrolysis has an impact on the average molecular weight (expressed in Dalton, Da) of the final product. The term "collagen hydrolysate" may be used interchangeably and synonymous with the terms "hydrolysed collagen" or "collagen peptide". The "collagen hydrolysate" in the context of the current invention encompasses collagen which is subjected to hydrolysis or partial hydrolysis. The collagen hydrolysate can be one or more of enzyme-hydrolysed, alkali-hydrolysed and acid-hydrolysed collagen hydrolysate. In addition or alternatively, the collagen hydrolysate can be obtained by one of the following processes or a combination thereof: alkali hydrolysis, acid hydrolysis and enzyme hydrolysis. The "collagen hydrolysate" in the context of the current invention may be produced from a collagen-containing material in a one-step process or via an intermediate gelatin stage (i.e. thus "hydrolysed gelatin" is obtained). The term "collagen hydrolysate" thus encompasses hydrolysed gelatin (i.e. hydrolyzed gelatin). The term "gelatin" in the context of the current invention means a (irreversible) form of collagen as obtained by partial hydrolysis of collagen. Depending on the process used, two types of gelatin, namely type A (acid hydrolysis) and type B (alkaline hydrolysis) are generally obtained. In the context of the current invention, the "gelatin" can refer to either Type A or Type B gelatin, or a combination thereof. Depending on the physical and chemical methods of the partial hydrolysis, the molecular weight of the peptides can fall within a broad range (e.g. 10-95 kDa). The partial hydrolysis provides the gelatin the ability to hold water and its gelling capacity, typically distinguishing it from "collagen peptide" or "collagen hydrolysate " i.e. products obtained by full hydrolysis of collagen. The term "hydrolysed gelatin" in the context of the current invention means a product obtained by the hydrolysis of gelatin, and which leads to a lower molecular weight of the gelatin. The hydrolysis reaction as to obtain hydrolyzed gelatin involves the rupturing of one or more of the peptide linkings with the addition of 1 molecule of water. Hydrolyzed gelatin distinguishes from gelatin among others, in terms gelling capacity, i.e. hydrolyzed gelatin has reduced/no gelling capacity and gelatin has gelling capacity. The gelatin may be hydrolyzed by acids (hydrogen ions), i.e. to obtain "acid-hydrolyzed gelatin". The gelatin may be hydrolyzed by alkali (hydroxyl ions), i.e. to obtain "alkali-hydrolysed gelatin". The gelatin may be hydrolyzed by one or more enzymes (e.g. pepsin, trypsin), i.e. to obtain "enzyme-hydrolysed gelatin".

The "collagen" or "collagen hydrolysate" in the context of the current invention may include proteins or peptides that are synthesized artificially, such as recombinantly or by chemical synthesis. Recombinant synthesis encompasses that a protein or peptides is encoded by recombinant DNA that is expressed in an expression system. The expression system for the recombinant peptide can be cells such as a bacterial cell (e.g., Escherichia coli, Bacillus subtilis species), yeast cell [e.g. Saccharomyces cerevisiae, Pichia pastoris or Ogataea angusta (Hansenula polymorpha), Candida bodini], fungal cell (e.g. Aspergillus oryzae, Aspergillus niger, Trichoderma reesei), mammalian cell (e.g. a CHO cell, a HeLa cell, a HEK293 cell, NS0, Sp2/0), insect cell or plant cell (e.g. tobacco, cereal, legume, fruit, vegetable). Synthetic synthesis encompasses one or more of solid phase synthesis, chemoenzymatic synthesis, Merrifield synthesis and Bailey peptide synthesis.

In various embodiments of the current invention, the collagen hydrolysate may be produced by enzymatic hydrolysis or partial enzymatic hydrolysis of collagen, wherein the enzyme used for this purpose may be one or more selected from the group consisting of serine protease, alkaline protease, neutral protease, flavor protease, complex protease, thiol protease, bromelain, metalloprotease, protease, carboxypeptidase, pepsin, chymotrypsin, trypsin, cathepsin K, chymotrypsin, papain and subtilisin. In an embodiment, the hydrolysis, preferably the enzymatic hydrolysis, is performed at pH 5-8, preferably pH 6-7. In an embodiment, the hydrolysis, preferably the enzymatic hydrolysis, is performed at a temperature of 55-70 °C, preferably 60-65 °C. In an embodiment, the hydrolysis, preferably the enzymatic hydrolysis, is performed for 3-8 hours, preferably 4-7 hours, more preferably 5-6 hours.

The collagen hydrolysate in the context of the current invention may be derived from one or more types of collagen selected from collagen types I - XXVII, preferably one or more of type I, II, III, V, or X collagen, more preferably one or more of type I, II or III collagen. In addition or alternatively, the collagen hydrolysate in the context of the current invention may be a mixture of two or more types of collagen, preferably two one or more of type I, type II, and type III collagen.

The collagen and/or collagen hydrolysate as disclosed herein may be derived from any one or more animals or species of animals, such as bovine (species), pig (species), chicken and fish (species). In an embodiment, the collagen is derived from a cow. In an embodiment, the collagen as is derived from a pig. In an embodiment, the collagen is derived from a fish. In an embodiment, the collagen is derived from a chicken. In various embodiments, the collagen is a mixture of collagen from different sources, such as collagen originating from multiple animal species and/or collagen originating from different tissues. For example, the collagen as disclosed herein may be a mixture of two or more collagens chosen from the group consisting of fish collagen, porcine collagen, chicken collagen, and bovine collagen.

The collagen in the context of the current invention may be derived from one or more tissues selected from the group consisting of skin, scale, antler, protrusions (e.g. humps) horns, head, brain, neck, ear, eye, nose, tongue, lip, mouth, oesophagus, trachea, sternum, larynx, bronchi, limbs, feet, toes, palms, claws, bones, cartilage, bone marrow, joints, membranes, hind, ligaments, tendon, rib, diaphragm, muscle, skeletal muscle, smooth muscle, intestine, blood vessels, bladder, stomach, aorta, heart, liver, kidney, chest, lung, spleen, pancreas, egg, sperm, testis, ovary, nerve, gallbladder and belly. The term "skin" as disclosed herein encompasses "hide", i.e. meaning the outer covering of large animals such as from bovine (species) or any other large animals. The terms "skin" and "hide" may herein be used interchangeably, and may refer the outer coverage of an animal, irrespective of size.

In a preferred embodiment, the collagen as taught herein is derived from skin and/or skin connective tissue. In preferred embodiment, the collagen as taught herein is derived from cartilage. In a preferred embodiment, the collagen as taught herein is derived from bone. In a preferred embodiment, the collagen as taught herein is derived from sternum. The collagen as disclosed herein may be a mixture of collagens derived from two or more tissues and/or two or more animals. In an embodiment, the collagen as disclosed herein is a mixture of two or more collagens selected from the group consisting skin collagen, cartilage collagen, sternum cartilage and bone collagen.

It is considered that amelioration sleep can be achieved with collagen hydrolysates from different sources and in a wide range of molecular weights, e.g. in the range of 1000-9000 Da (which is typical for collagen hydrolysates). The proof that different collagen hydrolysates can be suitable for ameliorating sleep was found by executing a study with the aim to investigate the single-dose bioavailability of collagen hydrolysate from different sources (bovine, fish and porcine) and of different mean molecular weight (2000 Da and 5000 Da for bovine collagen hydrolysate). The study showed that the uptake of collagen hydrolysates in blood appears similar for collagen hydrolysates from the sources porcine, bovine and fish and also for collagen hydrolysates with a relatively high average molecular weight (5000 Da) or relatively lower average molecular weight (2000 Da).

The collagen hydrolysate may have an average molecular weight in the range of 500 Da - 25000 Da, e.g. 1000 Da - 15000 Da or 2000 Da - 10000 Da. The collagen hydrolysate may have an average molecular weight of at least 500 Da, or 600 Da, or 700 Da or 800 Da, or 900 Da, or 1000 Da or 1100 Da, or 1200 Da, or 1300 Da, or 1400 Da, or 1500 Da, or 1750 Da, or 2000 Da, or 2250 Da, or 2500 Da, or 2750 Da, or 3000 Da, or 3250 Da, or 3500 Da, or 4000 Da, or 4500 Da, or 5000 Da, or 5500 Da. In addition or alternatively, the collagen hydrolysate may have an average molecular weight of no more than 10000 Da, or 9500 Da, or 9000 Da, or 8750 Da, or 8500 Da, or 8250 Da, or 8000 Da, or 7750 Da, or 7500 Da, or 7250 Da, or 7000 Da, or 6750 Da, or 6500 Da, or 6250 Da, or 6000 Da, or 5750 Da, or 5500 Da, or 5250 Da, or 5000 Da, or 4500 Da, or 4000 Da, or 3500 Da, or 3000 Da, or 2500 Da.

It appears most beneficial in the context of the current invention and for the improvement in overall sleep if the collagen hydrolysate has an average molecular weight above 1000 Da and below 6000 Da, in particular if in the range of 1500 - 3000 Da. In a preferred embodiment, the collagen hydrolysate has an average molecular in the range of 500 - 7500 Da, more preferably 1000 - 6000 Da, even more preferably 1500-3000 Da.

The average molecular weight in the context of the current invention is preferably the weight-average molecular weight (Mw).

A preferred way of measuring the (average) molecular weight and/or molecular weight distribution of the collagen hydrolysate is by high performance size exclusion chromatography (HPSEC). The following protocol is a preferred HPSEC protocol:
The Agilent HPLC, 1260 Infinity series (G1316A, G1329B, G1311C, G1315D) with a TSKgel SWXL precolumn und a G2000SWXL column (Tosoh Bioscience) is used. Analysis is performed with the WinGPC software (PSS). The eluent is 100 mM phosphate buffer pH 5.3. Samples are eluted from the column (e.g. 0.5 mL/min, isocractic) and monitored with UV detection (e.g. 214 nm, analysis time: 40 min per injection + 180 min equilibration). Calibration is performed with the Narrow Calibration Standard (Low FILK).

### Use of collagen hydrolysate in relationship to other active ingredients

In an embodiment, the composition for the use according to the invention does not contain any other active ingredients in addition to the collagen hydrolysate.

In certain embodiments of the present disclosure, the collagen hydrolysate may be combined for the use disclosed herein with one or more other active ingredients, for example that have a general positive effect on health or performance (such as vitamins, minerals, fibres etc.)

In an embodiment of the present disclosure, the composition comprises one or more other active ingredients in addition to the collagen hydrolysate, wherein the active ingredient preferably is not a compound capable of modulating sleep.

### Use of collagen hydrolysate in relationship to other compounds capable of modulating sleep

Without being bound by theory, it is found that collagen hydrolysate has an inherently strong sleep-enhancing effect. Although the exact reason is still unknown, the present inventors find that the sleep-enhancing effect of collagen hydrolysate may be counteracted by other known sleep-enhancing compounds, in particular gamma-aminobutyric acid (GABA), L-Theanine, 5-Hydroxytryptophan, and/or (L-)Tryptophan. As such, the collagen hydrolysate is preferably administered to an individual without further sleep-enhancing compounds.

In an embodiment of the present disclosure, the collagen hydrolysate is used in absence of a further compound and/or active ingredient capable of modulating sleep. In an embodiment of the present disclosure, the collagen hydrolysate is administered in a composition consisting (essentially) of collagen hydrolysate and/or comprising (essentially) no further compound and/or active ingredient capable of modulating sleep.

In an embodiment of the present disclosure, the use disclosed herein pertains to use of a composition comprising collagen hydrolysate and essentially no GABA or no more than 1 wt.%, or 0.1 wt.% or 0.01 wt.% GABA, calculated on the total amount of active ingredients and/or the compounds capable of modulating sleep.

In an embodiment of the present disclosure, the use disclosed herein pertains to use of a composition comprising collagen hydrolysate and essentially no melatonin or no more than 1 wt.%, or 0.1 wt.% or 0.01 wt.% melatonin, calculated on the total amount of active ingredients and/or the compounds capable of modulating sleep.

In an embodiment of the present disclosure, the use disclosed herein pertains to use of a composition comprising collagen hydrolysate and essentially no L-theanine or no more than 1 wt.%, or 0.1 wt.% or 0.01 wt.% L-theanine, calculated on the total amount of active ingredients and/or the compounds capable of modulating sleep.

In an embodiment of the present disclosure, the use disclosed herein pertains to use of a composition comprising collagen hydrolysate and essentially no 5-hydroxytryptophan or no more than 1 wt.%, or 0.1 wt.% or 0.01 wt.% 5-hydroxytryptophan, calculated on the total amount of active ingredients and/or the compounds capable of modulating sleep.

In an embodiment of the present disclosure, the use disclosed herein pertains to use of a composition comprising collagen hydrolysate and essentially no tryptophan or no more than 1 wt.%, or 0.1 wt.% or 0.01 wt.% tryptophan, calculated on the total amount of active ingredients and/or the compounds capable of modulating sleep.

The term "essentially consisting of collagen hydrolysate" or "essentially comprising no further compound" (than collagen hydrolysate) in the context of the current disclosure means that a further substance or compound other than collagen hydrolysate may be present, but that said further substance or compound is not measurable according to a standard technique in the field, and/or is below a certain threshold, preferably below 0.1 wt.%, more preferably below 0.01 wt.%, even more preferably below 0.001 wt.%, most preferably below 0.0001 wt.%, such as calculated on the total weight or the total active ingredients in a composition.

The collagen hydrolysate of the invention is preferably a compound capable of modulating sleep, preferably improving sleep. The term "further compound capable of modulating sleep" means any compound capable of modulating sleep that is not collagen hydrolysate.

When referring to collagen hydrolysate as a "compound" in context of the current disclosure, this means the total mix of amino acid chains (e.g. peptides) the collagen hydrolysate is composed of. For instance, if a certain composition comprises 10 wt.% collagen hydrolysate, this means that the total mix of amino acid chains (e.g. peptides) of the collagen hydrolysate is 10 wt.% in the composition.

For instance, if a composition comprises 10 wt.% collagen hydrolysate and 10 wt.% GABA (as only two active ingredients in the composition), then it is considered that the composition comprises 50 wt.% collagen hydrolysate and 50% GABA, as calculated on the compounds capable of modulating sleep in the composition.

The terms "compound capable of modulating sleep" or "active ingredient capable of modulating sleep" in the context of the current disclosure mean a compound, or mixture of compounds, that have been demonstrated to have an effect on ameliorating sleep, e.g. in terms of improving one or more of sleep onset (time), sleep time, sleep quality, sleep pattern and cognitive function (after sleep) and/or ameliorating daytime sleepiness and/or awakenings after sleep onset. A "compound capable of modulating sleep" encompasses compounds or mixture of compounds acting on the sleep-wake cycle and/or influencing the activity of neurotransmitters, hormones, and/or by binding to the specific receptors that regulate the sleep-wake cycle. Examples of "compounds capable of modulating sleep" in the context of the current disclosure include gamma aminobutyric acid (GABA), GABA receptor agonists (e.g. a benzodiazepine, baclofen, pregabalin, Vigabatrin), enhancers of endogenous GABA receptor agonists (e.g. a GABA analog, topiramate, gabapentin, Tiagabine, Valproic acid, phenobarbital), melatonin, precursors of melatonin (e.g. (L-)tryptophan), melatonin receptor agonists (Rozerem, Valdoxan, Hetlioz, 6-chloromelatonin, 2-(2-phenylethyl)benzofuran), Luzindole, 4-Phenyl-2-propionamidotetralin, enhancers of endogenous melatonin (e.g. 5-HTP, L-theanine, magnesium, vitamin B6, valerian or valerian root), serotonin, precursors of serotonin (e.g. (L-tryptophan)), serotonin receptor agonists (e.g. Lysergic acid diethylamide, Psilocybin, Buspirone, Sumatriptan), enhancers of endogenous serotonin (e.g. Fenfluramine, Lexapro, Fluoxetine, Sertraline), glucocorticoid inhibitors, antagonists of glucocorticoid receptors, inhibitors of endogenous glucocorticoid, and anti-insomnia agents. The enhancer of endogenous serotonin in the context of the current disclosure is preferably a selective serotonin re-uptake inhibitor such as one or more of selected from the group consisting of fluoxetine, sertraline, paroxetine, fluvoxamine, citalopram, escitalopram, vilazodone, vortioxetine, dapoxetine and nefazodone.

In the context of the current disclosure, the "compounds capable of modulating sleep" are preferably "sleep-modulating compounds" in the composition, meaning that the compounds have a demonstrated effect in modulating sleep as part of the composition of the invention. For example, GABA is sleep-modulating compound in the composition, if a modulation of sleep (e.g. improvement of sleep) by GABA is measured by a suitable method as disclosed herein. In this example, this can for example be done comparing the effect of a composition with GABA or without GABA (as a control) on sleep.

An "enhancer" of an endogenous compound in the context of the current disclosure means that the levels of said compound are increased when the "enhancer" is administered (e.g. orally), wherein the increase is preferably by at least 5%, more preferably at least 25%, even more preferably at least 50%, most preferably at least 100% and/or is a statistically significant measurable increase. For example, it is known that L-theanine intake enhances the GABA levels in the body. L-theanine is therefore an example of an enhancer of endogenous GABA in the context of the current disclosure.

The compound capable of modulating sleep can be derived from a plant and/or can be a plant extract. For example, melatonin is also found in certain plants and can therefore be a compound derived from plants and/or be a plant extract in the context of the current disclosure. A compound capable of modulating sleep that is derived from plant and/or is a plant extract may for example be selected from the group consisting of melatonin, valerian (root), chamomile, lavender, passionflower, lemon balm, skullcap, kava, horsetail (Equisetum), tocotrienol, resveratrol (3,5,4'-trihydroxy-trans-stilbene), cannabidiol, astaxanthin, beta-carotene, Camu Camu (Myrciaria dubia), hemp and ashwagandha.

In a preferred embodiment of the present disclosure, the compound and/or active ingredient capable of modulating sleep is 5-Hydroxytryptophan (i.e. 5-HTP). 5-HTP is a precursor to serotonin, a neurotransmitter that plays a key role in regulating sleep. When 5-HTP is taken as a supplement, it is converted to serotonin in the body, which can then help to promote sleep.

In a preferred embodiment of the present disclosure, the compound and/or active ingredient capable of modulating sleep is GABA or a GABA analog.

In a preferred embodiment of the present disclosure, the compound and/or active ingredient capable of modulating sleep is L-theanine. L-theanine is an amino acid that is commonly used as a dietary supplement to promote relaxation and improve sleep. L-theanine is thought to work by increasing the levels of GABA, serotonin, and dopamine in the brain, which are neurotransmitters that play a key role in regulating sleep.

In a preferred embodiment of the present disclosure, the compound and/or active ingredient capable of modulating sleep is (L-)tryptophan. (L-)Tryptophan is an essential amino acid that is a precursor to serotonin, a neurotransmitter that plays a key role in regulating sleep. When tryptophan is taken as a supplement, it is converted to 5-HTP in the body, which is then converted to serotonin and eventually melatonin.

In a preferred embodiment of the present disclosure, the compound and/or active ingredient capable of modulating sleep is melatonin. Melatonin is a hormone that is thought to regulate the body's internal clock and plays a key role in sleep. It is produced by the pineal gland in the brain and its levels increase in the evening as darkness falls and decrease in the morning with light exposure. Melatonin is thought to help regulate the sleep-wake cycle and promote feelings of drowsiness and sleepiness.

In a preferred embodiment of the present disclosure, the compound and/or active ingredient capable of modulating sleep is an anti-insomnia agent.

The term "anti-insomnia agent" in the context of the current disclosure means a compound, preferably a drug, used to treat insomnia, including but not limited barbiturates such as secobarbital, amobarbital, butalbital, cyclobarbital, pentobarbital, allobarbital, barbital, butobarbital, mephobarbital, phenobarbital, butabarbital, and vinylbital; quinazolinones and derivatives such as cloroqualone, diproqualone, etaqualone (Aolan, Athinazone, Ethinazone), mebroqualone, mecloqualone (Nubarene, Casfen), and methaqualone (Quaalude); benzodiazepines such as clorazepate, diazepam, flurazepam, halazepam, prazepam, lorazepam, lormetazepam, oxazepam, temazepam, clonazepam, flunitrazepam, nimetazepam, nitrazepam, adinazolam, alprazolam, estazolam, triazolam, climazolam, loprazolam, midazolam; non-benzodiazepines, such as zaleplon and eszopiclone; antihistamines such as diphenhydramine and doxylamine; melatonin; antidepressants such as trazodonee, amitriptyline, doxepin, trimipramine, mianserin and mirtazapine; antipsychotics such as chlorpromazine, clozapine, olanzapine, quetiapine, risperidone, zotepine, clonidine, guanfacine, cannabidiol, tetrahydrocannabinol, suvorexant, pregabalin, gabapentin, phenibut, imagabalin. The term "anti-insomnia agent" encompasses any agent that that requires prescription by a medical healthcare professional and/or sleep specialist.

### Administration of the collagen hydrolysate

In a preferred embodiment, the collagen hydrolysate as disclosed herein is administered orally.

The terms "administer" or "administration" in the context of the current invention encompass providing a compound, substance, or composition to a subject consuming it. A subject consuming a compound, substance, or composition may administer it to himself/herself. In such a case the term "administer" can be read as "take in". The term "administering" includes self-administering.

Without being bound to theory, the present inventors found an optimal bioavailability of the collagen hydrolysate and its derived components after oral intake relatively close to bedtime, and which is associated with improved sleep (e.g. as compared to earlier oral intake).

It is most beneficial in the context of the current invention and for overall improvement in sleep if the (daily dose of) collagen hydrolysate is administered relatively close to bedtime, such as 4 h - 15 min before bedtime, more preferably 2 h - 30 min before bedtime. In a preferred embodiment, the (daily dose of) collagen hydrolysate is administered within 4 h, or 3 h, or 2 h, or 2.5 h, or 1 h, or 45 min, or 30 min, or 15 min prior to bedtime.

The term "bedtime" means the moment that an individual goes to bed to sleep, or goes to bed anticipating to sleep, irrespective of the time of sleep onset.

The term "daily dose" in the context of the current invention means the total dry weight amount administered to a subject per day.

The term "unit dose" in the context of the current invention means the total dry weight amount administered to a subject in a single dose. The unit dose typically is in a pre-prepared form (e.g. prepacked dosage) ready for administration. The unit dose may for example (also) be identifiable from the product packaging or label. Examples of unit dosage forms are individual tablets, pre-filled sachets, individual capsules, bulk powders, liquid solutions, emulsions or suspensions.

It is most beneficial in the context of the current invention and overall improvement in sleep if a relatively low dose of collagen hydrolysate is administered, e.g. no more than 50 g, preferably no more than 30 g, even preferably no more than 20 g, most preferably no more than 15 g. In a preferred embodiment, the use of collagen hydrolysate as disclosed herein comprises administering the collagen hydrolysate in an amount of 2-50 g, preferably 5-30 g, even more preferably 10-20 g, wherein the dose is the unit dose and/or the daily dose, preferably the daily dose. Without being bound by theory, the present inventors found that a relatively low dose of collagen hydrolysate allows ameliorating sleep without providing unnecessary calories and/or promoting weight gain, which can be considered particularly for individuals not necessitating weight increase and/or individuals that are not physically active.

In different embodiments, the daily dose of the collagen hydrolysate is at least 1 g, 2 g, 3 g, 4 g, 5 g, 10 g, 15 g, 20 g, 25 g , 30 g, 35 g, 40 g, 45 g, or 50 g, wherein the daily dose is the total dry weight amount administered to a subject per day. In addition or alternatively, in different embodiments, the daily dose of the collagen hydrolysate as disclosed herein is no more than 50 g, 45 g, 40 g, 35 g, 30 g, 25 g, 20 g, 15 g, 14 g, 13 g, 12 g, 11 g, 10 g, 5 g, or 1 g, wherein the daily dose is the total dry weight amount administered to a subject per day.

It is most beneficial in the context of the current invention and for overall improvement in sleep if the daily dose of collagen hydrolysate is administered as a single unit dose, and preferably relatively close to bedtime as disclosed herein.

The present inventors found that the overall sleep-enhancing effects of collagen hydrolysate occurred relatively soon when administered without other additives that are known to enhance sleep, in particular without GABA receptor agonists and/or melatonin receptor agonists, and when the collagen hydrolysate is administered sufficiently frequently, such as at least once every other day or more preferably daily. In addition or alternatively, the effects of collagen hydrolysate in overall improvement in sleep are seen quickest if administered for 4 consecutive days, in particular 7 consecutive days (in a row).

In an embodiment, the collagen hydrolysate is administered every day or every other day.

In an embodiment, the collagen hydrolysate is administered at least once a day.

In an embodiment, the dosage regimen for collagen hydrolysate comprises administering collagen hydrolysate for at least 2 consecutive days, preferably for at least 4 consecutive days, more preferably for at least 7 consecutive days.

In an embodiment, the collagen hydrolysate is administered for at least 1 consecutive week, preferably for at least 2 consecutive weeks, more preferably for at least 4 consecutive weeks.

The terms "consecutive" or "consecutively" used in the context of collagen hydrolysate administration means that the administrations follow one another in order without gaps in a given time period. For example, when "collagen hydrolysate is administered for 4 consecutive days", this means that the collagen hydrolysate is administered for at least once a day for 4 days in a row (e.g. on Monday, Tuesday, Wednesday, and Thursday of the same week), irrespective of the number of administrations per day or the total number of administrations. For example, when "collagen hydrolysate is administered for 4 consecutive weeks", this means that the collagen hydrolysate is administered for at least once a week for 4 weeks in a row, irrespective of the number of administrations per day, the number of administrations per week or the total number of administrations.

### Collagen hydrolysate formulation

The collagen hydrolysate in the context of the current invention may be provided in a food formulation, food supplement formulation, or pharmaceutical formulation, preferably a food supplement formulation.

The collagen hydrolysate as disclosed herein may be provided in a solid dosage form such as a capsule, a tablet, or a powder, preferably a powder.

The collagen hydrolysate as disclosed herein may be provided in a formulation such as a drinkable solution or suspension, drink such as beer, syrup, artificially-flavoured drink, carbonated beverage, (water-soluble) powdered mixture, (water-soluble) paste, (water-soluble) powder, (water-soluble) tablet, (water-soluble) pill, (water-soluble) dragee, (water-soluble) caplet, (water-soluble) sachet, or (water-soluble) capsule. In addition or alternatively, the collagen hydrolysate as taught herein may be present in a functional food, e.g. a juice, shake, dairy drink, yoghurt, yoghurt drink, dessert, energy bar, nutritional bar, slimming bar, or confectionery such as gummies or center-filled gummies.

### Use in physically active individuals

The present inventors found that the collagen hydrolysate of the current invention is particularly effective in improving overall sleep in physically active individuals, and in particular athletes. Very physically active individuals, and in particular athletes, may suffer from poor sleep or even insomnia-related symptoms like day-time fatigue or daytime sleepiness. It appears that such physically active individuals can benefit greatly from administration of collagen hydrolysate according to the invention.

In a preferred embodiment, the collagen hydrolysate is administered to a physically-active individual.

In a preferred embodiment, the collagen hydrolysate is administered to an individual performing exercise regularly, e.g. at least once a week, or at least twice a week, or at least three times a week, or at least four times a week, or at least five times a week, or at least six times a week, or at least seven times a week and/or 1-10 times a week, or 2-7 times a week, or 3-5 times a week. In addition or alternatively, in a preferred embodiment, the collagen hydrolysate is administered to an individual performing a minimal volume of exercise, e.g. at least 2 h per week, or at least 4 h per week, or at least 6 h per week, or at least 8 h per week, or at least 10 h per week, or at least 12 h per week, or at least 14 h per week, or at least 16 h per week, or at least 18 h per week, or at least 20 h per week and/or 1-24 h per week, or 3-20 h per week, or 5-16 h per week, or 7-14 h per week.

The term "exercise" in the context of the current invention means any physical activity that can be planned, structured, and has a final or an intermediate objective (e.g. the improvement or maintenance of physical fitness). The term "exercise" encompasses all professional or non-professional activities including: sports, strength or resistance training, and endurance or aerobic activities. In addition or alternatively, "exercise" may include endurance training. The term "endurance training" refers to training the aerobic system as opposed to the anaerobic system, e.g. by performing physical exercise at an increased heart rate for a prolonged period of time (e.g. at 70% maximum heart rate (MHR) for at least 30 minutes), for example during walking, running, jogging, cycling, swimming, rope jumping etc.

In an embodiment, a physically active individual is defined according to an at least moderate or average physical activity score, preferably a high physical activity score, such as according to a physical activity questionnaire (PAQ). The PAQ is preferably one or more of:
(i) International Physical Activity Questionnaire-Short form (IPAQ-SF), which is generally considered the most frequently used and validated PAQ (IPAQ-SF for example reported by Ahmad et al. Baltic Journal of Health and Physical Activity 2015; 7(1): 29-41);
(ii) Global Physical Activity Questionnaire (GPAQ), designed by the World Health Organization (WHO) for chronic disease risk factor surveillance and is considered the most widely used PAQ internationally;
(iii) European Health Interview Survey-Physical Activity Questionnaire (EHIS-PAQ), an EU-wide surveillance system of all member state.
(Semper et al. Validity and Reliability of International Physical Activity Questionnaires for Adults across EU Countries: Systematic Review and Meta Analysis. Int. J. Environ. Res. Public Health 2020, 17, 7161)

For instance, an individual scored as performing at least "moderate" physical activity preferably falls into the categorical score of 2 or 3 using the IPAQ-SF. In the IPAQ-SF, the following categorical score are used:
1. Low: no activity is reported, or some activity is reported but not enough to meet Categories 2 or 3.
2. Moderate: either of the following 3 criteria (i) 3 or more days of vigorous activity of at least 20 minutes per day, (ii) 5 or more days of moderate-intensity activity and/or walking of at least 30 minutes per day, (iii) 5 or more days of any combination of walking, moderate-intensity or vigorous- intensity activities achieving a minimum of at least 600 Metabolic Equivalent Task (MET)-minutes/week.
3. High: any one of the following 2 criteria (i) Vigorous-intensity activity on at least 3 days and accumulating at least 1500 MET-minutes/week, (ii) 7 or more days of any combination of walking, moderate- or vigorous-intensity activities accumulating at least 3000 MET minutes/week.

In a preferred embodiment, the collagen hydrolysate is administered to an athlete. In an embodiment, the "athlete" is an individual fulfilling at least three, preferably all, of the following criteria:
1) the individual is performing exercise aiming to improve his/her performance/results;
2) the individual participates in a sport competition;
3) the individual is formally registered in a local, regional or national sport federation;
4) the individual has exercise training and sports as his/her major activity or focus of personal interest, devoting several hours in all or most of the days for these activities, exceeding the time allocated to other types of professional or leisure activities.

### Sleep-enhancing effects of the collagen hydrolysate

In an embodiment, the collagen hydrolysate is used for ameliorating, preferably decreasing sleep onset time. In an embodiment, the collagen hydrolysate is used for ameliorating, preferably increasing, sleep time (after sleep onset). In an embodiment, the collagen hydrolysate is used for ameliorating sleep pattern (after sleep onset). In an embodiment, the collagen hydrolysate is used for ameliorating, preferably increasing, sleep quality (after sleep onset). In an embodiment, the collagen hydrolysate is used for ameliorating, preferably decreasing, awakenings after sleep. In an embodiment, the collagen hydrolysate is used for ameliorating, preferably decreasing, daytime sleepiness.

Improvements in sleep can be determined using parameters the skilled person is familiar with, such as one or more of Sleep Onset Latency (SOL), Wake After Sleep Onset (WASO, e.g. min or h), Total Sleep Time (TST, e.g. min or h), Sleep Efficiency (SE, %), number of awakenings (nWAK), number of awakenings per hour of sleep (WAKI), time in bed (min), total sleep time (min or h), sleep latency N2 (min or h), deep sleep latency (min or h), total sleep period (min or h), sleep stage change (count), sleep stage change (count/hr), REM stage sleep# (%), N1 stage sleep# (%), N2 stage sleep# (%), N3 stage sleep# (%), night sleep# (%), arousal (count), arousal (count/hr). Sleep may additionally or alternatively be assessed according to the number of nights requiring sleep medication to support sleep, improvements in daytime functioning (e.g. assessed by the Work and Social Adjustment Scale, improvements in various quality of life measures (such as with the 36- Item Short Form Survey, SF-36) and/or reductions in daytime sleepiness (e.g. assessed by Karolinska Sleepiness Scale or the Epworth Sleepiness Scale)

In an embodiment, the collagen hydrolysate is used for ameliorating, preferably decreasing, awakenings after sleep, preferably as determined by polysomnography (PSG).

The term "sleep pattern" in the context of the current invention means the time spent in each of the sleep stages, including the relative proportion of the duration of each stage as compared to the duration of other stages, as measured, for example, by spectral analysis of electroencephalograms. Parameters of "normal" and "abnormal" sleep patterns for specific populations are well known to the skilled person (see, for example, Latta et al., 2005, Sleep 28:1525-1534). In mammals, sleep is typically divided into two broad types, rapid eye movement (REM) and non-rapid eye movement (non-REM) sleep. Non-REM can be further divided into three stages, N1, N2, and N3 (or delta or slow-wave sleep). Sleep proceeds in cycles of REM and Non-REM. There is a greater amount of deep sleep (stage N3) earlier in the sleep cycle, while the proportion of REM sleep increases later in the sleep cycle and just before natural awakening. In humans, each sleep cycle lasts on average from 90 to 110 minutes. REM sleep, accounts for 20-25% of total sleep time in most adults. The criteria for REM sleep include rapid eye movements as well as a rapid low- voltage EEG. In the context of the current invention, an amelioration of sleep pattern encompasses an increase in the amount, frequency and/or duration of REM sleep. The sleep pattern of a subject may be monitored overnight using electrodes and an electroencephalograms (EEGs), or a (wearable) device designed to monitor and measure various physiological parameters.

In an embodiment, the collagen hydrolysate is used for ameliorating, preferably decreasing, daytime sleepiness.

The amelioration of sleep in the context of the current invention encompasses improving, preferably decreasing, daytime sleepiness, more preferably sleepiness in the morning or afternoon. The daytime sleepiness can be measured with the Karolinska Sleepiness Scale (KSS). The KSS measures the subjective level of sleepiness at a particular time during the day. The KSS has been used in studies of shift work, jetlag, for driving abilities. The KSS spans 9 levels (1 = extremely alert, 2 = very alert, 3 = alert, 4 = rather alert, 5 = neither alert nor sleepy, 6 = some signs of sleepiness, 7 = sleepy, but no effort to keep awake, 8 = sleepy, some effort to keep awake, 9 = very sleepy, great effort keeping awake, fighting sleep). In the context of the current invention, the KSS measurement is preferably started in the morning after sleep and repeated throughout the morning and afternoon, e.g. every hour. An amelioration of sleep can for instance be defined according to a decrease in KSS score by one or more points at one or more time points.

In an embodiment, the collagen hydrolysate is used for ameliorating, preferably increasing, cognitive function, wherein the cognitive function is preferably the cognitive function after sleep. In an embodiment, the collagen hydrolysate is used for ameliorating, preferably decreasing, cognitive impairment, preferably the cognitive impairment after sleep.

The term "after sleep" in the context of the current invention preferably means in the morning and/or the first 4 hours (e.g. first 3 hours, first 2 hours, first hour, first 30 min) after the normal sleep time. For example, if an individual normally sleeps until 9 AM, then the time "after sleep" is the period until 1 PM. For example, if an individual may normally sleep until 6 AM, then the time "after sleep" is the period until 10 AM.

In the context of the current invention, the cognitive function can be established according to one or more of a reaction time test, a choice reaction time test, a digit span test and a Stroop (color & word) test and/or any suitable test used to determine cognitive impairment, such as the Montreal Cognitive Assessment (MoCA).

The reaction time can for example be determined by asking participants to press the spacebar on a keyboard when a green circle appears on a blank screen, wherein the reaction time can be the fastest or the average time (in ms or s).

The choice reaction time can for example be determined by asking participants to press the arrow key that matches the direction on the screen, wherein the choice reaction time can be the fastest or the average time (in ms or s).

The digit span test (in arbitrary units) can for example involve asking participants to type the digits in the correct order corresponding to a sequence of 3-14 digits flashed on a screen. The Stroop test can for instance involve measuring a baseline level, for example when participants are presented with names of colours (written in black) on a white background and are instructed to correctly identify the name of the colour by pressing the right of left arrow. The Stroop test can for instance involve measuring an interference level, for example when participants are presented with colored names on a white background and are instructed to correctly identify the font color, ignoring the incongruent colour named on the screen (e.g., if "green" is written in red font, then red it the correct answer). In the Stroop test, participants are instructed to respond as quickly as possible using left or right arrow keys and the outcome are the proportion of correct responses and/or or the average reaction time of correct responses (in ms or s), for the baseline and interference levels.

In an embodiment, the collagen hydrolysate is used for ameliorating, preferably increasing, cognitive function (after sleep), wherein amelioration of cognitive function is preferably established according to a Stroop test, more preferably as according to a Stroop color & word test.

In an embodiment, the collagen hydrolysate is used for inhibiting cognitive interference (after sleep), wherein the inhibition in cognitive interference is defined according to a Stroop test, more preferably as according to a Stroop color & word test.

The term "amelioration" in the context of the current invention means an improvement, such as a relative to a control or reference (population) not receiving the collagen hydrolysate of the invention and/or an improvement compared to the situation prior to initiation of administration of the collagen hydrolysate of the invention. The improvement can be determined for example by measurements made daily, and average measures for a week may be compared to average measure prior to initiation of the treatment, or average measure of a control or reference (population), to determine improvements in sleep. The improvement can be an at least 1% improvement, such as at least 5%, at least 10%, at least 15%, at least 20%, at least 25%, at least 30%, at least 35%, at least 40%, at least 45%, or at least 50% improvement, compared to a corresponding level in a control or reference and/or compared to a level prior to initiating administration of the collagen hydrolysate of the invention. In addition or alternatively, a level can be considered to be improved when it is significantly improved based on an appropriate statistical test, e.g. compared to a level in a control or reference and/or compared to a level prior to initiating administration of the collagen hydrolysate of the invention. An amelioration can mean an improvement in one or more of "sleep onset time", "awakenings after sleep", "sleep time", "sleep quality", "sleep pattern", "daytime sleepiness", "cognitive function after sleep", "daytime functioning" and a "quality of life measure" as disclosed herein. An amelioration can mean an amelioration in cognitive function, preferably cognitive function after sleep, e.g. using a suitable test as disclosed herein. An amelioration encompasses a reduction in the score in a cognitive impairment test such as the Montreal Cognitive Assessment (MoCA). An amelioration of sleep can mean a decrease of the AIS from a value of 6 or higher (i.e. indicating presence of insomnia), to a value of below 6 (i.e. indicating absence of insomnia). An amelioration of sleep can mean a decrease in AIS by at least 1 point, such as a decrease by 1,2,3,4,5,6,7,8,9,10,11,12,13,14,15,16,17,18,19,20, 21,22,or 23 points. An amelioration of sleep can mean a decrease in KSS by at least 1 point, such as a decrease by 1,2,3,4,5,6,7, or 8 points.

In a preferred embodiment, the collagen hydrolysate improves one or more selected from the group consisting of sleep onset time, sleep time, sleep quality, sleep pattern and cognitive function after sleep.

In a preferred embodiment, the collagen hydrolysate ameliorates awakenings after sleep onset and/or daytime sleepiness.

### Individuals benefiting from an improvement in sleep

The collagen hydrolysate of the invention is suitable for any individual benefiting from an improvement in sleep and/or desiring an improvement in sleep, including individuals suffering a sleep disorder, individuals suffering chronic lack of sleep, or without (chronic) sleep problems. With the term "lack of sleep" a sleep duration is meant less than the average sleep requirement of the individual concerned. The use of the collagen hydrolysate can be considered either therapeutic or non-therapeutic depending on the context and subject concerned. A professional medical practitioner and/or sleep specialist is able to determine on a case-by-case basis whether lack of sleep and/or a desire of improvement in sleep is a therapeutic or a non-therapeutic intervention (in other words, whether a person falls in the group of healthy subjects or in the group of pathological subjects. Therapeutic use encompasses a use in in a pathological situation, such as in case of a sleep disorder and/or chronic lack of sleep. Therapeutic use is usually in an individual having sleep-related symptoms of pain and suffering and/or wherein lack of sleep may lead to (serious) health and psychological risks, which can be established for example by a professional medical practitioner and/or sleep specialist. Therapeutic use is typically in an individual receiving a sleep-enhancing drug, such as an anti-insomnia agent, e.g. following consultation of a professional medical practitioner and/or sleep specialist. Non-therapeutic use encompasses use in a non-pathological context, i.e. in "sleep-healthy" individual, such as in an individual not suffering from morbid sleep, but may benefit from an improvement in sleep such as when there is a lack of sleep and/or when there is a desire for more sleep to improve general well-being. In "sleep-healthy" individuals, the lack of sleep may have a nature or severity such that it naturally disappears over time (i.e. it is not chronic). In addition or alternatively, in "sleep-healthy" individuals, the lack of sleep may be such that he/she would generally not seek help from a professional medical practitioner and/or sleep specialist. The collagen hydrolysate in the context of the current invention may offer one or more of the following advantages in "sleep-healthy" individuals: improve the general performance, improve cognitive function (e.g. attention, concentration, memory), increasing productivity in daily life, increasing the (cosmetic) appearance.

In an embodiment, the use of collagen hydrolysate of the invention is therapeutic. In an embodiment, the use of collagen hydrolysate of the invention is non-therapeutic, e.g. cosmetic and/or for improving general well-being or functioning. In an aspect, the current disclosure describes a method of treatment, involving administrating the collagen hydrolysate to an individual experiencing lack of sleep.

### Use in sleep disorders

In an aspect, the present disclosure describes collagen hydrolysate and/or a composition comprising the collagen hydrolysate, for use in ameliorating a sleep disorder.

The use in a sleep disorder is preferably a therapeutic use.

In an aspect, the current present disclosure describes a method of ameliorating, such as preventing and/or treating, a sleep disorder, wherein the method involves administering to an individual the collagen hydrolysate and/or a composition comprising the collagen hydrolysate.

In an embodiment, the sleep disorder is insomnia. The skilled person is aware of the different types of insomnia and their classifications (Fietze et al. Front Psychiatry. 2021 Jun 29;12:683943). The term "insomnia" in the context of the current invention encompasses acute insomnia and chronic insomnia. "Chronic insomnia" in the context of the current invention is disrupted sleep that occurs at least three nights per week and lasts at least three months. "Acute insomnia" in the context of the current invention is insomnia that is not chronic insomnia.

The preferred method of determining whether an individual has insomnia is with the Athens Insomnia Scale (AIS). The AIS is measured by assessing eight factors (sleep induction, awakenings during the night, final awakening, total sleep duration, sleep quality, well-being during the day, functioning capacity during the day, sleepiness during the day) which are rated on a 0-3 scale and the sleep is finally evaluated from the cumulative score of all factors and reported as an individual's sleep outcome. A cut-off score of ≥6 on the AIS is used to establish the diagnosis of insomnia (Soldatos et al. Journal of Psychosomatic Research, 48(6), 555-560).

In an embodiment, presence of insomnia is defined by an AIS of 6 or more, such as 6-24, or 12-24, or 18-24.

Insomnia and similar sleep disorders may be alternatively or additionally diagnosed by clinical interview with a psychologist, and/or may include the completion of self-reporting questionnaires and sleep diaries. Questionnaires suitable for determining presence of insomnia in the context of the current invention is the Insomnia Severity Index (Bastien et al., 2001, Sleep Med 2:297-307) and the Insomnia Symptom Questionnaire (Spielman et al., 1987, Sleep 10:45- 56).

In an embodiment, the sleep disorder is restless legs syndrome. The presence of restless legs syndrome may be determined by a professional medical practitioner and/or a sleep specialist, such as according to the criteria as established by the International Restless Legs Syndrome Study Group:
a) a strong, often irresistible urge to move the legs, usually accompanied by uncomfortable sensations,
b) symptoms start or get worse when resting, such as sitting or lying down,
c) symptoms are partially or temporarily relieved by activity, such as walking or stretching,
d) symptoms are worse at night,
e) symptoms can't be explained solely by another medical or behavioral condition.

In an embodiment, the sleep disorder is narcolepsy. The presence of narcolepsy may be determined by a professional medical practitioner and/or a sleep specialist such as based on one or more of sleep history, sleep record, sleep study (e.g. polysomnography), multiple sleep latency test, genetic tests and a lumbar puncture.

In an embodiment, the sleep disorder is sleep apnoea. The presence of sleep apnoea may be determined by a professional medical practitioner and/or a sleep specialist such as based on a nocturnal polysomnography and/or a home sleep test.

### Collagen hydrolysate-based compositions

In an embodiment of the present disclosure, the composition (for use) comprises one or more further compounds capable of modulating sleep.

In a preferred embodiment of the present disclosure, the composition (for use) pertains to a composition comprising at least 60 wt.%, preferably at least 65 wt.%, more preferably at least 70 wt.%, even more preferably at least 75 wt.% collagen hydrolysate as active ingredient, calculated on the total weight of active ingredients and/or the total weight of compounds capable of modulating sleep.

In a preferred embodiment of the present disclosure, the composition (for use) disclosed herein pertains to a composition comprising at least 90 wt.%, preferably at least 95 wt.%, more preferably at least 97.5 wt.%, even more preferably at least 99 wt.% collagen hydrolysate as active ingredient, calculated on the active ingredients and/or the compounds capable of modulating sleep in the composition, wherein the composition may comprise one or more further compounds capable of modulating sleep.

In an embodiment of the present disclosure, the composition, such as for the use disclosed herein, consists (essentially) of collagen hydrolysate, calculated on the active ingredients and/or the compounds capable of modulating sleep in the composition.

In an embodiment, the composition, such as for the use disclosed herein, comprises (essentially) no further compound and/or active ingredient capable of modulating sleep. In an embodiment, the composition, such as for the use disclosed herein, comprises at least 20, 30, 40, 50, 60, 65, 70, 75, 80, 85, 90, 91, 92, 93, 94, 95, 96, 97, 98, 99, 99.9, 99.8, 99.7, 99.6, 99.5, 99.4, 99.3, 99.2, 99.1, 99.99, or 100 wt.% collagen hydrolysate, calculated on the active ingredients and/or the compounds capable of modulating sleep in the composition.

In an embodiment, the composition, such as for the use disclosed herein, comprises at least 0.01, 0.05, 0.1, 0.2, 0.3, 0.4, 0.5, 0.6, 0.7, 0.8, 0.9, 1, 2, 3, 4, 5, 6, 7, 8, 9, 10, 15, 20, 25, 30, 35, 40, 50, 60, 70 or 80 wt.% of one or more further compounds and/or active ingredients capable of modulating sleep.

In an embodiment , the present disclosure pertains to a composition, suitable for the use disclosed herein, comprising at least 70 wt.%, preferably at least 80 wt.%, more preferably at least 97.5 wt.%, even more preferably at least 99 wt.% collagen hydrolysate as active ingredient and one or more further compounds capable of modulating sleep, calculated on the total weight of active ingredients and/or the compounds capable of modulating sleep in the composition.

In an embodiment, the composition (for use) of the invention is a sleep-ameliorating composition. In an embodiment, the composition (for use) of the invention is suitable for ameliorating sleep. In an embodiment, the composition of the invention is suitable for ameliorating one or more of awakenings after sleep onset, sleep time, sleep quality, sleep pattern, daytime sleepiness, and cognitive function after sleep.

In various embodiments, the current invention pertains to a use of the collagen hydrolysate as disclosed herein, involving administering the collagen hydrolysate in a (sleep-enhancing) composition as disclosed herein.

### General definitions

The terms 'comprising' or to comprise' and their conjugations are used in the context of the current invention in their non-limiting sense to indicate that items following the word are included, but items not specifically mentioned are not excluded.

Reference to an element by the indefinite article 'a' or 'an' does not exclude the possibility that more than one of the elements is present, unless the context clearly requires that there be one and only one of the elements. The indefinite article 'a' or 'an' thus usually means 'at least one'.

In the context of the current invention, a level is considered as "increased" or "decreased" when it is at least 1% higher or lower, respectively, than the corresponding level in a control or reference (for example 5%, 10%, 15%, 20%, 25%, 30%, 35%, 40%, 45%, or 50% higher or lower). In addition or alternatively, a level in a sample may be considered increased or decreased when it is statistically significantly higher or lower, respectively, compared to a level in a control or reference (including compared to an earlier time point), irrespective of the size of the change. The term "to reduce" may in the context of the current invention be used interchangeably with the term "to decrease".

### FIGURE LEGENDS

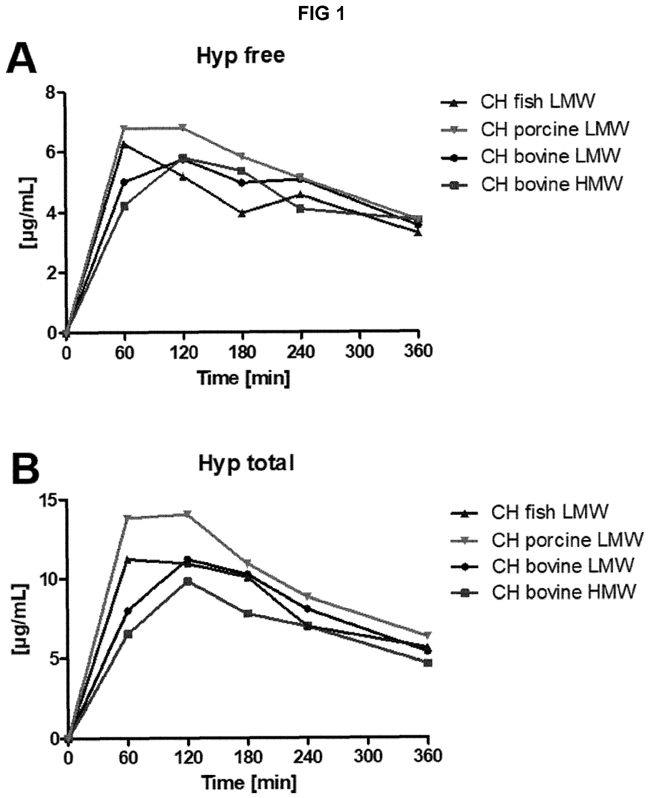

**Figure 1****.** Concentration-time curves of the free Hyp response [µg/mL] (A) or total Hyp response [µg/mL] (B) after intake of study products (mean, SD); n=6. CH=collagen hydrolysate; LMW=low molecular weight; HMW=high molecular weight.

### EXAMPLES

### Example 1

Example 1 examines the effects of collagen hydrolysate (CH) administration on sleep in males with sleep complaints.

### Methods

### Sample size estimation

The sample size was determined from an *a priori* power analysis (G*power 3.1.9.2, Microsoft Windows) for two primary outcome measures: sleep onset latency (SOL), and sleep efficiency (SE), as measured by polysomnography (PSG). A mean difference of 10 min (10 min SD) for SOL, and 4% for SE (4% SD) was used to estimate the sample size. With two tails, and an alpha of 0.05, it was calculated ≥10 participants are needed to achieve 80% statistical power.

### Participants

Thirteen athletic males with sleep complaints provided written informed consent to participate in this study. De characteristics of the participants are presented in Table 1. Height and body mass were recorded at familiarisation. The inclusion criteria were: 1) males 18-35 years; 2) Athens Insomnia Scale (AIS) score ≥6, indicating difficulty sleeping; 2) morningness-eveningness (MEQ) score of 31-69, indicating neither extreme morning or evening person; 3) scheduled exercise ≥3 occasions per week in the past 6 months. Participants completed a health history questionnaire and were excluded if they used sleep medications, had a diagnosed sleep disorder, reported excessive use of substances that negatively affect sleep such as drugs, alcohol, or smoking, regularly used anti-inflammatory medications (within 2 weeks of participation), had a food allergy, or any other contraindication to the study procedures.

**Table 1- Physical characteristics and activity levels of study participants.**

| **Variable** | ***Mean* ±*SD*** |
|---|---|
| **Age (years)** | 24 ± 4 |
| **Height (m)** | 1.8 ± 0.1 |
| **Body mass (kg)** | 79.2 ± 12.6 |
| **AIS** | 9 ± 2 |
| **MEQ** | 50 ± 8 |
| **Exercise training frequency (n/week)** | 5 ± 2 |
| **Exercise training volume (h/week)** | 7 ± 3 |

| | |
|---|---|
| AIS, Athens Insomnia Scale; MEQ, morningness-eveningnessquestionnaire. | |

### Experimental design

Prior to the supplementation trials, participants completed a familiarisation trial, in which they had their physical activity and sleep monitored for 7 days and nights in their home. This was to characterise exercise and sleep schedules (i.e., bedtimes and wake times) for the supplementation trials, and to familiarize them with the protocol and equipment. No supplements were consumed for these 7 days. Sleep was monitored via actigraphy and subjective sleep quality for 7 days, and then PSG on the final 2 nights (nights 6 and 7). Following this familiarisation trial, participants were randomised, in a double-blind manner, to consume 15 g CH (collagen hydrolysate) or a placebo (PLA) in 200 ml 1 h before bedtime for 7 days (trials were separated by ≥7 days). CH was provided for 7 days as this duration of intake has previously been shown to positively influence physiological markers.

Sleep monitoring during both trials was the same as the familiarisation trial. Surveys to assess fatigue and sleepiness were also completed daily. On day 7, participants reported to the lab in the evening (≥16:00), had a venous blood and urine sample collected, consumed a telemetric temperature pill (E-celcisus Performance Pill, Body Cap, France) and performed a battery of cognitive function tests. Participants returned to the lab after an overnight fast on the morning of day 8 (09:00 - 10:00) to have another blood and urine sample collected and to repeat the cognitive function tests. Participants were instructed to keep their bedtime and wake times the same as on their familiarisation week. On day 7 of both trials, participants followed a low protein diet and consumed a standardised evening meal. No exercise was permitted on day 7. Participants were instructed to replicate their dietary intake and physical activity patterns recorded during the familiarisation week.

### Administrations

Participants consumed 15 g/day of CH derived from bovine hide (molecular weight ~2000 Da) or an inert, taste matched, placebo control as a 200 ml liquid bolus, without food, 1 h before their scheduled bedtime (as recorded in the familiarisation trial) for 7 days. Both supplements were provided in identical opaque bottles by Rousselot BV (Ghent, Belgium). The investigators remained blinded until data analysis was completed. No participants reported adverse effects from the supplements.

### Athens insomnia scale and morning-eveningness questionnaire

The AIS is an eight-item survey that assesses sleep difficulty in the past month; a score of ≥6 is indicative of poor sleep (Soldatos et al. Journal of Psychosomatic Research, 48(6), 555-560). The AIS is based on diagnostic criteria from the International Classification of Diseases Tenth Revision (ICD-10) and has excellent reliability and validity in adult populations. It also has excellent reliability in athletic populations. The MEQ measures circadian rhythms, differentiating individuals into morning and evening types. A score of <31 indicates an extreme evening type; a score of 69 indicates an extreme morning type. Extreme morning or evening types were excluded to increase the homogeneity of the sample.

### Sleep and training diaries, and actigraphy

Participants used sleep diaries to self-report their time in bed, lights out, SOL, number of awakenings, length of awakenings, wake up time, and get up time during the trials. Any day-time naps were also recorded. Training diaries were used to record the intensity and volume of scheduled exercise completed each day. Intensity (1 = very low intensity, 10 = maximum intensity) was multiplied by duration (min) to give a composite score of training load. Participants also wore wristwatch actigraphs (ActiGraph GT9X Link, Actigraph, FL, US) on their non-dominant arm to measure physical activity levels and sleep. The participants were instructed not to remove the watch apart from to shower or bathe; if the watch was removed at any other point, they were instructed to record this in their sleep diaries. Actigraphy data was sampled at 30 second epochs and analyzed using the Cole Kripke algorithm (Cole et al., 1992) on ActiLife software (ActiGraph, FL, US). Lights out, wake up and get up times from the sleep diaries were used for data analysis. Sleep variables analyzed from actigraphy were total time in bed (TIB), total sleep time (TST), SOL, SE, wake after sleep onset (WASO) and average wake length. Physical activity was analysed as total time (min) spent in moderate to vigorous activity using the cut off points suggested by Freedson and colleagues (Medicine & Science in Sports & Exercise, 30(5), 777-781).

### Polysomnography

Polysomnographic sleep was measured on nights 6 and 7 of each trial with an ambulatory monitor (Embletta MPR with ST+ Proxy, Natus Medical, CA, US). Night 6 served as a familiarization collection only and was not used for analysis. Six electroencephalogram (EEG) channels (F3-M2; F4-M1; C3-M2; C4-M1; O1-M2 and O2-M1), two electrooculogram (EOG) channels (LEOG-M2 and REOG-M1), a submental chin electromyogram (EMG) (3 EMG electrodes) and the PSG unit were fitted in the lab ~6-8 hours before sleep. All EEG sites were located using the international 10-20 electrode placement. The PSG was scheduled to start recording 30 min prior to their bedtime. All recordings were collected at home; participants were instructed to sleep in the same bed, in the same environmental conditions and expose themselves to the same mental stimulation (e.g., phone use, television) in the 1 h before bed on each trial. Any deviances were reported in their subjective sleep diaries. Home-based PSG is considered as valid and reliable as clinic-based PSG set ups. Recordings were subsequently exported in EDF format and downloaded into Domino software v3.0.0.3 (SOMNOmedics, Germany) where they were manually scored in 30 second epochs using the standard adult criteria from the American Academy of Sleep Medicine (AASM) ("AASM Scoring Manual - American Academy of Sleep Medicine," n.d.) by registered sleep physiologists with ≥20 years' experience. Each recording was labelled with a different non-identifiable code so that the scorers (RNK and KA) were blinded to participant identity and trial. As with actigraphy, lights off was marked as the time participants first attempted to go to sleep and lights on was marked as the time participants woke up for the final time (both from sleep diaries). To assess intrarater reliability, 3 blinded PSG recordings were scored twice by RNK. To assess interrater reliability, KMA independently scored 3 PSGs previously scored by RNK. Each recording was scored for the outcomes: time in bed (min), total sleep time (min), sleep latency (min), sleep efficiency (%), sleep latency N2 (min), deep sleep latency (min), REM sleep latency (min), total sleep period (min), sleep stage change (count), sleep stage change (count/hr), wakening's (count), wakening's (count/hr), wake after sleep onset (min), REM stage sleep# (%), N1 stage sleep# (%), N2 stage sleep# (%), N3 stage sleep# (%), night sleep# (%), arousal (count), arousal (count/hr).

### Subjective surveys

Subjective sleep quality was recorded with a 7-point Likert scale (where 1 = extremely poor and 7 = extremely good). Sleepiness was measured with the Karolinska Sleepiness Scale (KSS, where 1 = extremely alert, and 10 = extremely sleepy, can't stay awake). Fatigue was measured with a 100 mm visual analogue scale (where 0 mm = not fatigued at all and 100 mm = extremely fatigued). Sleep quality was recorded upon waking, while KSS and fatigue scores were recorded upon waking, at 14:00 and 1 h before bed. These were recorded daily with 7-day averages used for analysis.

### Cognitive function

Participants completed a series of cognitive tests that took approximately 10 minutes on a laptop. All tests were preceded by 3-5 practice trials. The first test was simple reaction time (RT), where participants were instructed to press the spacebar when a green circle appeared on a blank screen. The second test was choice RT, where participants were presented with right or left arrows and were required to press the arrow key that matched the direction on the screen. Average and fastest reactions times of correct responses were analyzed for both; there were 10 and 14 attempts for simple and choice RT, respectively. Participants then completed the digit span test, which measures attention and short-term recall. To complete this test, a sequence of 3-14 digits were flashed on the screen and then participants were instructed to type the digits in the correct order. The length of the longest correct span over 12 trials was used for analysis. Finally, participants completed the Stroop color word test, which measures attention and processing speed, but primarily the ability to inhibit cognitive inference. For the baseline level, participants were presented with names of colours (written in black) on a white background and instructed to correctly identify the name of the colour by pressing the right of left arrow. For the interference level, participants were presented with colored names on a white background and were required to correctly identify the font color, ignoring the incongruent colour named on the screen (e.g., if "green" is written in red font, then red it the correct answer). Participants had to respond as quickly as possible using left or right arrow keys. Outcomes were the proportion of correct responses, and average RT of correct responses (ms) and for the baseline (14 trials) and interference (20 trials) levels.

### Dietary control

Participants recorded their dietary intake on day 7 of the familiarization trial and were instructed to replicate this intake on the two supplementation trials. On this day they were instructed to limit their intake of protein-rich foods (a list was provided), avoid consuming any caffeine after 11:00, and attend the lab ≥2 h post-prandial. Participants were given a low-protein evening meal to consume at home consisting of 1 x 250 g pack of White Golden Vegetable Rice (Tesco, PLC, Herts, UK) and 2 x 37 g Nutrigrain Blueberry bars (Kelloggs, Michigan, US). After this meal they avoided any food or fluids other than water and their respective supplements 1 h before sleep until after their lab visit on day 8.

### Blood and urine samples

Blood and urine samples were collected the evening of day 7 and the morning of day 8. Urine samples were collected in a plastic container; venous blood samples were obtained via venipuncture into 1 x 10 ml vacutainer for serum and 1 x 10 ml vacutainer coated with EDTA to obtain plasma. The EDTA vacutainer was immediately centrifuged at 3000 rpm (4° C) for 10 minutes, while the serum vacutainer was left to stand for 30 min prior to centrifugation. Urine samples and plasma and serum supernatants were subsequently stored in aliquots at - 80°. Samples were analyzed for high sensitivity c-reactive protein (hs-CRP), interleukin-6 (IL-6), cortisol, and noradrenaline, normetanephrine, as these are sensitive to changes in sleep quality (Faraut et al., 2012). IL-6 was analyzed in plasma using a commercially available ELISA kit (R and D Systems, MN, US); the inter-assay coefficient of variation (CV) was <6.9%.

### Measurements of serum cortisol, urine noradrenaline and normetanephrine by LC-MS/MS

Liquid chromatography tandem mass spectrometry (LC-MS/MS) methods were performed by a Waters Acquity I-class UPLC system coupled to the Xevo TQ-XS tandem mass spectrometer (Waters Corp., Milford, MA, USA) operated in positive electrospray mode. Serum cortisol was extracted using the Extrahera^{™} automation system (Biotage, Uppsala, Sweden) under positive pressure supplied by a nitrogen generator. In a 2mL well plate, 200 µL of calibration standards (Chromsystems, München, Germany NIST SRM971 traceable), quality control materials, and serum samples were added to each well with 50 µL internal standard solution containing cortisol-d4 (IsoSciences, King of Prussia, PA, USA). 200 µL of isopropanol:water 50:50 (v/v) was then added to dissociate the binding proteins. After mixing, the samples were loaded onto ISOLUTE^{®} supported liquid extraction (SLE+) 400 µL plate (Biotage). Elution was carried out by adding two cycles of 750 µL of methyl tertiary butyl ether (MTBE). The eluents were collected into a corresponding deep well plate. Positive pressure was applied at each stage to remove residual solvent. Samples were then dried to completeness under a gentle stream of nitrogen gas heated to 60°C, then reconstituted with 100 µL of 50:50 methanol/water before being vortexed and sealed. 10 µL of the extract was injected into the LC-MS/MS. Chromatographic separation was achieved using a CORTECS^{™} core-shell C18 50 × 2.1mm, 2.7 µm, reversed-phase (Waters Corp., Milford, MA, USA) column heated at 30°C. Mobile phases used were (A) water and (B) methanol in 0.1% formic acid, pumped at the flow rate of 0.4 mL/min in 70:30% (A:B), gradually increased to 100% (B) then returned to the starting gradient at 4 minutes. Tandem mass spectrometry detection was based on the mass-to-charge (m/z) precursor to product ion transitions specific to each compound: cortisol (363.3>121.2) and cortisol-d4 (367.3>121.2). The inter-assay CV for serum cortisol was <6.0% across the assay range of 0.3-806 nmol/L.

Urine noradrenaline and normetanephrine were extracted using solid phase extraction method (Chromsystems Biogenic amino #80600, München, Germany) as per the manufacturer's instruction. The assay was calibrated using human urine-based calibration standards and controls (Chromsystems) that are traceable to certified reference materials. m/z transitions were: noradrenaline (152.1>107.1), normetanephrine (166.1>106.1), noradrenaline-d6 (158.1>111.1) and normetanephrine-d3 (169.1>109.1). The inter-assay CV for noradrenaline was <6.7% across the assay range of 23.6-4421 nmol/L; normetanephrine CV was 3.4-6.2% across the assay range of 20.5-10311 nmol/L. Urine results obtained from LC-MS/MS analysis were adjusted for variations in renal function by dividing by urine creatinine. Urine creatinine was analysed using Roche 2nd generation kinetic colorimetric assay based on the Jaffé method performed on the COBAS^{®} C501 analyser (Roche, Burgess Hill, UK). The inter-assay CV was <2.1%. The final results are expressed as nmol/L per mmol/L creatinine. Data for noradrenaline are not presented due to a analytical difficulties with several samples.

### Serum C-Reactive Protein high sensitive (hs-CRP)

Serum hs-CRP was measured using a particle-enhanced immunoturbidimetric assay analysed on the COBAS^{®} C501 analyser (Roche, Burgess Hill, UK). The inter-assay CV was <2.6% across the assay working range 1.43-190 nmol/L.

### Data analysis

Data analysis was performed with SPSS (IBM SPSS Statistics for Windows, Version 27.0. Armonk, NY: IBM Corp). Normality was assessed by checking histograms and skewness and kurtosis of the residuals and homogeneity of variance by plotting the residuals against the predicted values. Variables collected via PSG and actigraphy, sleep quality, and physical activity were measured with paired t-tests; any variables not normally distributed were analysed with non-parametric Wilcoxon signed rank tests. Actigraphy and subjective sleep data is an average of the 7 supplementation nights. Core temperature, KSS, fatigue, and all cognitive function and blood outcomes were analysed with linear mixed models. Time, condition (CH vs. CON), and time*condition interaction effects were included as fixed factors, and participant as a random factor. Models were run using the keep it maximal approach; therefore, random intercepts and random slopes were included for all within-subject variables. The covariance type was scaled identity and the model estimation was restricted maximum likelihood. *Post hoc* tests were adjusted for multiple tests using the Bonferroni correction.

Intra and inter-rater reliability for PSG variables were assessed with intraclass correlation coefficients (ICC), using absolute agreement and a two way mixed-effects model (as in SPSS); values <0.50 were considered to have poor reliability, 0.50 -0.75 moderate reliability, 0.75 and 0.90 good reliability, and >0.90 excellent reliability. P<0.05 was considered statistically significant. Hedges g effect sizes are reported for between condition pairwise comparisons.

### Results

The main findings are that 7 days of CH supplementation improves overall sleep as compared to the placebo control. The most largest effects were found on 1) reduced awakenings, as measured by PSG and subjective sleep diaries, and 2) improved cognitive performance, as measured by a Stroop test.

Frequency of awakenings was significantly less in CH (1.3 ± 1.5 counts) vs. CON (1.9 ± 0.6 counts) (P = 0.025; g = 0.539). There was a time*condition interaction effect for correct responses on the baseline Stroop test (P = 0.007) with post-hoc tests revealing that during the CH trial participants responses were more accurate (P = 0.009; g = 0.573).

This study suggests CH could be used as a non-pharmacological strategy to enhance sleep in individuals with sleep complaints, in particular in physically active individuals such as athletes.

### Example 2

Example 2 examines the effects of different formulations based on collagen hydrolysate on recreationally active individuals with sleep complaints.

### Methods

Example 2 uses a similar experimental design as Example 1.

### Participants

The participants are recreationally active normal weight male adults (18-30 years) with sleep complaints, based on a threshold value of 6 of higher on the Athens Insomnia Scale (AIS).

### Formulations and administration

Beverages (volume 150 ml) are prepared based on the formulations according to Table 2. The CH and the placebo control are the same as in Example 1. The total amount of sleep-enhancing active ingredients or placebo is always 15 g.

The administration is daily 60 min before sleep for a period of 7 days.

**Table 2. Formulations tested for the effect on sleep parameters. The wt.% are calculated on the total of sleep-enhancing active ingredients in the formulation. Abbreviations: CH= collagen hydrolysate, GABA=gamma-aminobutyric acid.**

| **Formulation** | **Active ingredients** |
|---|---|
| **#1 (Placebo control)** | n/a |
| **#2 (Pure CH)** | 100 wt.% CH |
| **#3 (CH/GABA blend)** | 90 wt.% CH, 5 wt.% GABA |
| **#4 (CH/melatonin blend)** | 99.1 wt.% CH, 0.1 wt.% melatonin |
| **#5 (Reference product)** | 49 wt.% CH, 49 wt.% glycine amino acids, 2 wt.% L-theanine |
| **#6 (Reference product)** | 97.5 wt.% CH, 2.4% GABA, 0.1 wt.% melatonin |

### Bioavailability

The bioavailability of the collagen hydrolysate after oral administration was determined in plasma samples.

### Sleep parameters

The sleep parameters are measured according to Example 1.

The number of awakenings is measured by PSG and subjective sleep diaries.

The cognitive performance is measured by a Stroop test.

### Results

The highest overall improvement in sleep is seen when administering 100% collagen hydrolysate as active ingredient without other additives that are known to enhance sleep (Table 3).

The effect of 100% collagen hydrolysate is already measurable within a week, but this effect is delayed in combination with other additives.

Participants taking formulations #3-6 report more complaints related to fatigue, mood, or psychomotor and neurocognitive performance.

**Table 3. Effect of formulations (according to Table 2) on sleep parameters and overall improvement in sleep.**

| **Formulation** | **Improvement on sleep awakening** | **Improvemen t on cognitive function** | **Speed of benefits seen** | **Overall improvemen t in sleep** |
|---|---|---|---|---|
| **#1** | *None* | *None* | *n*/*a* | ***None*** |
| **#2** | *High* | *High* | *High* | ***High*** |
| **#3** | *High* | *Low* | *Average* | ***Average*** |
| **#4** | *High* | *Low* | *Average* | ***Average*** |
| **#5** | *High* | *Low* | *Average* | ***Average*** |
| **#6** | *High* | *Low* | *Average* | ***Average*** |

It is found that the overall improvement in sleep is associated with the amount of collagen hydrolysate in the composition (relative to other sleep enhancing compounds).

The plasma profile showed that oral collagen hydrolysate is preferably administered relatively close to bedtime, and that the bioavailability is associated with improved sleep.

### Example 3

Example 3 shows the effect of collagen hydrolysates obtained from different sources or with different molecular weights.

### Objective

To investigate the single-dose bioavailability of collagen hydrolysate from different sources and of different mean molecular weight.

It is considered that a similar bioavailability of collagen hydrolysates indicates a similar functionality, such as in ameliorating sleep.

### Methods

Bioavailability was assessed by evaluating the uptake of free and peptide-bound hydroxyproline (Hyp) as marker amino acid. A randomized, double blind, cross-over clinical study was performed with healthy volunteers.

A single-dose of 10 g collagen hydrolysate was provided at low mean molecular weight (2000 Da). For the bovine collagen hydrolysate, a single-dose of 10 g was provided of either low mean molecular weight (2000 Da, "LMW") or high mean molecular weight (5000 Da, "HMW'). Blood was sampled for analysis over a period of 6 hours after collagen hydrolysate ingestion.

### Results

Figure 1 shows the concentration-time curves of the free Hyp response (A) or total Hyp response (B) after intake of study products.

It was found that, by intake of 10 g of the collagen hydrolysate, free Hyp concentrations in plasma (ΔCₘₐₓ) were greatly increased with an average factor of 7.2 for fish, 9.9 for porcine and 6.2 for both bovine low molecular weight (LMW) and high molecular weight (HMW) collagen hydrolysate. With respect to the bioavailability of free Hyp, there were no significant differences between the incremental area under the curve (iAUC) of the investigated products. In addition, Hyp content in blood samples was determined after total hydrolysis. Significantly higher concentrations of total Hyp were determined comparing the iAUC of free and total Hyp. The total Hyp concentrations in plasma (ΔCₘₐₓ) were also greatly increased for either fish and porcine collagen hydrolysate, and both for bovine collagen hydrolysate LMW or bovine collagen hydrolysate HMW.

Overall, the results show that the uptake of collagen hydrolysates in blood appears similar for collagen hydrolysates from the sources porcine, bovine and fish and also for hydrolysates with a relatively high average molecular weight (5000 Da) and relatively lower average MW (2000 Da).

A similar approach was used to study Glycine and Proline, which showed maximum were reached between 60 - 130 min without significant difference between the uptake kinetics from the different collagen hydrolysate sources or molecular weight. Based on the absorption kinetics (AUCs, Cₘₐₓ and Tₘₐₓ), the intake of free amino acids is highly comparable between collagen hydrolysates of different animal sources and different molecular weights.

## Claims

1. Use of collagen hydrolysate in non-therapeutic ameliorating of sleep, wherein the collagen hydrolysate is administered in a composition consisting of collagen hydrolysate, calculated on the active ingredients in the composition.

2. Use according to claim 1, wherein the collagen hydrolysate is administered within 4 h, preferably within 2 h, prior to bedtime.

3. Use according to any claim 1 or 2, wherein the collagen hydrolysate is administered at a daily dose of 2-50 g, preferably at a daily dose of 10-20 g.

4. Use according to any one of the previous claims, wherein the collagen hydrolysate is administered every day or every other day, and/or wherein the collagen hydrolysate is administered for at least one week.

5. Use according to any one of the previous claims, wherein the collagen hydrolysate has an average molecular weight of 1000-7500 Da.

6. Use according to any one of the previous claims, wherein the collagen hydrolysate is administered in a physically active individual, preferably in an athlete.

7. Composition comprising collagen hydrolysate for use in ameliorating a sleep disorder, wherein the collagen hydrolysate is administered in a composition consisting of collagen hydrolysate, calculated on the active ingredients in the composition.

8. Composition for use according to claim 7, wherein the sleep disorder is selected from the group consisting of insomnia, restless legs syndrome, narcolepsy and sleep apnea.

9. Composition for use according to claim 7 or 8, wherein the collagen hydrolysate improves one or more selected from the group consisting of sleep onset time, sleep time, sleep quality, sleep pattern and cognitive function after sleep, and/or wherein the collagen hydrolysate ameliorates awakenings after sleep onset and/or daytime sleepiness.

10. Composition for use according to any one of claims 7-9, wherein the composition is administered as defined in any one of claims 2-4.

11. Composition for use according to any one of claims 7-10, wherein the collagen hydrolysate is administered in a physically active individual, preferably in an athlete.

## Patentansprüche

1. Verwendung von Kollagenhydrolysat bei der nicht-therapeutischen Verbesserung des Schlafs, wobei das Kollagenhydrolysat in einer Zusammensetzung verabreicht wird, die aus Kollagenhydrolysat besteht, berechnet auf Grundlage der Wirkbestandteile in der Zusammensetzung.

2. Verwendung gemäß Anspruch 1, wobei das Kollagenhydrolysat höchstens 4 Std., vorzugsweise höchstens 2 Std., vor dem Zubettgehen verabreicht wird.

3. Verwendung gemäß einem beliebigen der Ansprüche 1 oder 2, wobei das Kollagenhydrolysat in einer Tagesdosis von 2 bis 50 g, vorzugsweise in einer Tagesdosis von 10 bis 20 g, verabreicht wird.

4. Verwendung gemäß einem beliebigen der vorhergehenden Ansprüche, wobei das Kollagenhydrolysat täglich oder jeden zweiten Tag verabreicht wird und/oder wobei das Kollagenhydrolysat mindestens eine Woche lang verabreicht wird.

5. Verwendung gemäß einem beliebigen der vorhergehenden Ansprüche, wobei das Kollagenhydrolysat ein mittleres Molekulargewicht von 1000 bis 7500 Da aufweist.

6. Verwendung gemäß einem beliebigen der vorhergehenden Ansprüche, wobei das Kollagenhydrolysat einem körperlich aktiven Individuum, vorzugsweise einem Sportler, verabreicht wird.

7. Zusammensetzung, die Kollagenhydrolysat zur Verwendung bei der Linderung einer Schlafstörung umfasst, wobei das Kollagenhydrolysat in einer Zusammensetzung verabreicht wird, die aus Kollagenhydrolysat besteht, berechnet auf Grundlage der Wirkbestandteile in der Zusammensetzung.

8. Zusammensetzung zur Verwendung gemäß Anspruch 7, wobei die Schlafstörung aus der Gruppe ausgewählt ist, die aus Schlaflosigkeit, dem Restless-Legs-Syndrom, Narkolepsie und dem Schlafapnoe-Syndrom besteht.

9. Zusammensetzung zur Verwendung gemäß Anspruch 7 oder 8, wobei das Kollagenhydrolysat aus der Gruppe, welche aus dem Einschlafzeitpunkt, der Schlafdauer, der Schlafqualität, dem Schlafmuster und der kognitiven Funktion nach dem Schlaf, mindestens ein ausgewähltes verbessert und/oder wobei das Kollagenhydrolysat die Aufwachereignisse nach dem Einschlafen und/oder die Schläfrigkeit tagsüber verbessert.

10. Zusammensetzung zur Verwendung gemäß einem beliebigen der Ansprüche 7 bis 9, wobei die Zusammensetzung gemäß den Bestimmungen in einem beliebigen der Ansprüche 2 bis 4 verabreicht wird.

11. Zusammensetzung zur Verwendung gemäß einem beliebigen der Ansprüche 7 bis 10, wobei das Kollagenhydrolysat einem körperlich aktiven Individuum, vorzugsweise einem Sportler, verabreicht wird.

## Revendications

1. Utilisation d'hydrolysat de collagène dans l'amélioration non thérapeutique du sommeil, dans laquelle l'hydrolysat de collagène est administré dans une composition constituée d'hydrolysat de collagène, calculé sur la base des ingrédients actifs dans la composition.

2. Utilisation selon la revendication 1, dans laquelle l'hydrolysat de collagène est administré dans les 4 heures, de préférence dans les 2 heures, avant le coucher.

3. Utilisation selon l'une quelconque des revendications 1 ou 2, dans laquelle l'hydrolysat de collagène est administré à une dose quotidienne de 2-50 g, de préférence à une dose quotidienne de 10-20 g.

4. Utilisation selon l'une quelconque des revendications précédentes, dans laquelle l'hydrolysat de collagène est administré tous les jours ou tous les deux jours, et/ou dans laquelle l'hydrolysat de collagène est administré pendant au moins une semaine.

5. Utilisation selon l'une quelconque des revendications précédentes, dans laquelle l'hydrolysat de collagène a un poids moléculaire moyen de 1 000 à 7 500 Da.

6. Utilisation selon l'une quelconque des revendications précédentes, dans laquelle l'hydrolysat de collagène est administré à un individu physiquement actif, de préférence à un athlète.

7. Composition comprenant un hydrolysat de collagène pour une utilisation dans l'amélioration d'un trouble du sommeil, dans laquelle l'hydrolysat de collagène est administré dans une composition constituée d'hydrolysat de collagène, calculé sur la base des ingrédients actifs de la composition.

8. Composition pour une utilisation selon la revendication 7, dans laquelle le trouble du sommeil est choisi dans le groupe constitué par l'insomnie, le syndrome des jambes sans repos, la narcolepsie et l'apnée du sommeil.

9. Composition pour une utilisation selon la revendication 7 ou 8, dans laquelle l'hydrolysat de collagène améliore un ou plusieurs choisis dans le groupe constitué par l'heure d'endormissement, la durée du sommeil, la qualité du sommeil, le modèle de sommeil et la fonction cognitive après le sommeil, et/ou dans laquelle l'hydrolysat de collagène améliore les réveils après l'endormissement et/ou la somnolence diurne.

10. Composition pour une utilisation selon l'une quelconque des revendications 7 à 9, dans laquelle la composition est administrée comme défini dans l'une quelconque des revendications 2 à 4.

11. Composition pour une utilisation selon l'une quelconque des revendications 7 à 10, dans laquelle l'hydrolysat de collagène est administré à un individu physiquement actif, de préférence à un athlète.
